Europäisches Patentamt

European Patent Office

Office européen des brevets

(11) Publication number: **0 365 172**
A2

## EUROPEAN PATENT APPLICATION

(21) Application number: 89310085.9

(22) Date of filing: 03.10.89

(51) Int. Cl.5: **G01N 33/576** , //**C12N15/36, C12R1:865**

The applicant has filed a statement in accordance with Rule 28 (4) EPC (issue of a sample only to an expert). Accession number(s) of the deposit(s): FERM BP-1059.

The microorganism(s) has (have) been deposited with Fermentation Research Institute under number(s) FERM BP-1059.

The applicant has filed a statement in accordance with Rule 28 (4) EPC (issue of a sample only to an expert). Accession number(s) of the deposit(s): IFO 10206,FERM BP-1059,IFO 10426,FERM BP-1747

(30) Priority: 05.10.88 JP 251421/88

(43) Date of publication of application:
25.04.90 Bulletin 90/17

(84) Designated Contracting States:
AT BE CH DE ES FR GB GR IT LI LU NL SE

(71) Applicant: TAKEDA CHEMICAL INDUSTRIES, LTD.
3-6, Doshomachi 2-chome Chuo-ku
Osaka(JP)

(72) Inventor: Fujisawa, Yukio
31-104, 1 Mikagenakamachi 4-chome
Higashinada-ku
Kobe Hyogo 658(JP)
Inventor: Kuroda, Shun'ichi
210, 3-63, Toyosato 1-chome
Higashiyodogawa-ku Osaka 533(JP)
Inventor: Miyazaki, Takeshi
13-2, Higashitokiwadai 4-chome Toyono-cho
Toyono-gun Osaka 563-01(JP)

(74) Representative: Lewin, John Harvey et al
ELKINGTON AND FIFE Beacon House 113
Kingsway
London WC2B 6PP(GB)

(54) Assay for anti-pre S antibody.

(57) Disclosed are methods for determining antibodies against the pre-S regions contained in the L-protein and the M-protein in hepatitis B virus (HBV) env proteins by enzyme immunoassays, in which nonspecific reactions can be depressed by pre-treatment of serum with an antigenic protein produced by a unicellular organism and by contacting an antigenic protein produced by a unicellular organism, whereby all anti-pre-S1 and anti-pre-S2 antibodies in human sera and the sera of experimental animals can be highly sensitively determined without the use of radioactive materials.

EP 0 365 172 A2

## ASSAY FOR ANTI-PRE-S ANTIBODY

### BACKGROUND OF THE INVENTION

The present invention relates to an assay for an anti-pre-S antibody, more particularly to a method for detecting an antibody against the pre-S regions contained in the L-protein and the M-protein in hepatitis B virus (HBV) env proteins by an enzyme immunoassay.

The pre-S antigen - anti-pre-S antibody system is induced in sera of patients with acute hepatitis infected with HBV and developing the symptoms thereof, prior to the induction of the HBs antigen - anti - HBs antibody system, the HBc antigen - anti-HBc antibody system and the HBe antigen - anti-HBe antibody system [A. Robert Neurath et al., Nature 315, 154 (1985)]. Thus, the kind of role played by the pre-S antigen and the anti-pre-S antibody in respect to the completion of HBV infection, the mechanism by which the symptoms of type B hepatitis are developed, and the prevention thereof is important. Experiments conducted by the use of chimpanzees show that antisera against a pre-S2 peptide in the pre-S region neutralize HBV [A. Robert Neurath et al., Vaccine 4, 35 (1986)] and the development of the symptoms of acute hepatitis caused by HBV is prevented with a pre-S2 peptide vaccine [Y. Itoh et al., Proc. Natl. Acad. Sci. U.S.A. 83, 9174 (1986)]. Antibodies against a pre-S1 peptide in the pre-S region prevent the binding of HBV to cells derived from hepatoma [A. Robert Neurath et al., Cell 46, 429 (1986)]. As the importance of anti-S antibodies is thus recognized, it has been desired to develop a system by which anti-pre-S antibodies in sera of hepatitis B patients or individuals inoculated with a hepatitis B vaccine can be determined easily and highly sensitively.

When antibodies in human sera are detected by usual enzyme immunoassays, remarkably nonspecific reactions are often observed, which differ greatly from the case in which the sera of experimental animals are used. For this reason, when human sera are used, the assays must be carried out using highly diluted sera, which results in an insufficient sensitivity of the enzyme immunoassays in many cases. Consequently, when a low level of antibody titer in human sera is measured, radioimmunoassays having higher sensitivity have been employed. The assays of anti-pre-S antibodies are also dependent on the radioimmunoassays. For example, an anti-pre-S antibody in sera of patients with acute type B hepatitis has been detected by detecting the antibody bound to a pre-S peptide in a solid phase by the radioimmunoassay in the antiglobulin method [A. Robert Neurath et al., Nature 315, 154 (1985)]. An anti-pre-S antibody in human sera has been detected by the inhibition method by allowing human sera to react with an HBs antigen solution containing a definite amount of pre-S antigen, then binding an unreacted pre-S antigen-containing HBs antigen to beads coated with an anti-pre-S antibody, and detecting it by an $^{125}$I-labeled anti-HBs antibody [A. Budkowska et al., Hepatology 6, 360 (1986)]. Further, with respect to a human antibody against a polymerized albumin receptor (PAR) in the pre-S2 region, an anti-PAR antibody has been determined by the inhibition method by allowing sera to react with PAR-containing HBs particles in a solid phase, then binding polymerized human serum albumin (poly- HSA) to an unreacted PAR, and detecting it by an $^{125}$I- labeled monoclonal anti-poly HSA antibody [H. Okamoto et al., Hepatology 6, 354 (1986)].

Thus, the radioimmunoassay is the only method known for assaying sensitively the anti-pre-S antibodies in human sera, even where assay synthetic peptides are frequently used as antigens. However, the radioimmunoassay has many restrictions from the viewpoint of safety and operability due to the use of radioactive materials, and has the disadvantage of it being impossible to detect all anti-pre-S antibodies due to the use of the synthetic peptides. There has thus been a need for a highly sensitive assay system in which non-radioactive materials are used and by which all induced anti-pre-S antibodies can be detected.

### SUMMARY OF THE INVENTION

The present inventors undertook to develop a system by which substantially all, preferably all, anti-pre-S antibodies in human sera and sera of experimental animals can be determined highly sensitively and simply without the use of radioactive materials.

Assays for determining the antibody titer highly sensitively without the use of radioactive materials include enzyme immunoassays (EIA) and enzyme linked immunosorbent assays (ELISA). When the anti-pre-S antibodies in human sera are detected by conventional EIAs or ELISAs, nonspecific reactions are observed. In order to prevent them, it is necessary to use sera diluted 100 to 400 times and synthetic peptides as antigens.

It has been discovered that the nonspecific reactions can be depressed by a pre-treatment of adding

2

previously S-protein or M-protein to sera, and there have been proposed an assay for determining anti-pre-S1 antibody which comprises first adding small spherical particles containing pre-S2 to sera to absorb anti-S antibodies and anti-pre-S2 antibody, then primarily reacting the resulting mixture with a tubular particle-derived polypeptide (S + M + L), and further reacting the resulting product with a labeled secondary antibody, and an assay for determining anti-pre-S2 antibody which comprises adding small spherical particles containing S protein alone to sera to absorb anti-S antibody, and then carrying out the primary reaction with polypeptide (M + S) enriched with the small spherical particles-derived middle-sized polypeptide and further similarly conducting the secondary reactions [E. Takai et al., Journal of Immunological Methods 95, 23-30 (1986)]. However, these assays have the disadvantage that the purification of the small spherical particles, the small spherical particles carrying pre-S2 and the tubular particles is difficult, because they are isolated from the sera of patients positive to the HBs antigen, which results in complicating the assays themselves and decreased assay accuracy.

The present inventors have developed an assay using S-, M- and L-proteins particles obtained from unicellular organisms by gene engineering procedures in place of the small spherical particles, the tubular particles-derived polypeptides and the polypeptides enriched with the small spherical particles-derived middle-sized polypeptide obtained from sera. As a result, highly sensitive enzyme immunoassays can be established by which substantially all, preferably all, anti-pre-S1 and anti-pre-S2 antibodies in human sera and sera of experimental animals can be determined individually or simultaneously.

According to the present invention, there is provided

(1) an immunoassay for an antibody against a pre-S2 region of a hepatitis B virus surface antigen in a serum which comprises mixing the serum and a hepatitis B virus surface antigen S-protein produced by a unicellular organism and bringing the mixture into contact with a hepatitis B virus surface antigen M-protein produced by a unicellular organism and bound to a carrier, thereby determining the amount of the antibody present against the pre-S2 region of the hepatitis B virus surface antigen bound to the carrier,

(2) an immunoassay for an antibody against a pre-S1 region of a hepatitis B virus surface antigen in a serum which comprises mixing the serum and a hepatitis B virus surface antigen M-protein produced by a unicellular organism and bringing the mixture into contact with a hepatitis B virus surface antigen L-protein produced by a unicellular organism bound to a carrier, thereby determining the amount of the antibody present against the pre-S1 region of the hepatitis B virus surface antigen bound to the carrier, and

(3) an immunoassay for an antibody against a pre-S region of a hepatitis B virus surface antigen in a serum which comprises mixing the serum and a hepatitis B virus surface antigen S-protein produced by a unicellular organism and bringing the mixture into contact with a hepatitis B virus surface antigen L-protein produced by a unicellular organism and bound to a carrier, thereby determining the amount of the antibody present against the pre-S region of the hepatitis B virus surface antigen bound to the carrier.


DESCRIPTION OF THE PREFERRED EMBODIMENT

The S-, M- and L-proteins may be isolated from sera of individuals positive to the HBs antigen. However, their purification is difficult. Hence, one preferably uses proteins produced by gene engineering procedures [P. Valenzuela et al., Nature 298, 347 (1982); A. Miyanohara et al., Proc. Natl. Acad. Sci. U.S.A. 80, 1 (1983); M. L. Michel et al., Proc. Natl. Acad. Sci. U.S.A. 81, 7708 (1984); Y. Fujisawa et al., Gene 40, 23 (1985); Y. Itoh & Y. Fujisawa, Biochem. Biophys. Res. Commun. 141, 942 (1986); P. Dehoux et al., Gene 48, 155 (1986); Japanese Patent Unexamined Publication No. 63-109795 (1988); M. Kobayashi et al., J. Biotech. 8, 1 (1988)]. As the S-protein, there may be used a protein obtained by removing the pre-S region from the L- or M-protein by protease degradation. For example, a protease, such as V8 protease (Seikagaku Kogyo) derived from Staphylococcus aureus V8 strain is suitable.

When the L-, M- and S-proteins are prepared by gene engineering procedures, animal cells or yeasts are used as preferred hosts in order to retain S-antigenicity. However, other host cells can be used. Yeasts such as Saccharomyces cerevisiae are especially preferable. The S- or M- protein produced from animal cells or yeasts, or mixtures thereof is preferably employed, as the S-protein used for absorption of anti-S antibodies contained in sera in assays for anti-pre-S antibodies (anti-pre-S1 antibody + anti-pre-S2 antibody) or the anti-pre-S2 antibody, and as the M-protein used for absorption of the anti-pre-S2 antibody and the anti-S antibodies contained in sera in assays for the anti-pre-S1 antibody.

As the L- and M-proteins to be bound to the carrier, there may be used proteins lower in S-antigenicity than the proteins used for the above-mentioned absorption. For example, there may be used proteins produced from procaryotes such as Escherichia coli (which proteins are very low in S- antigenicity) in addition to the L- and M-proteins produced from cells such as yeasts. When the M-protein is produced from animal cells, the resultant protein has the disadvantage of being small in the amount of the pre-S2 region bound to the carrier, because the particles are usually composed of the S-protein and the M-protein. Further, the L-protein is produced in low amounts from animal cells by secretion, and therefore it is not preferable to prepare the L-protein using animal cells as hosts.

As the above-mentioned S- and M-proteins to be used for the absorption of the anti-S antibodies and the like, and as the M- and L-proteins to be bound to the carrier, proteins produced from yeasts such as Saccharomyces cerevisiae are preferably employed.

When trypsin-like protease-producing yeast is used as a host, the L- and M- proteins are likely to be decomposed by the protease. It is therefore preferable to modify the proteins so as to delete arginine positioned at the 48th position from the N-terminus of the M-protein or a peptide containing it, preferably a peptide from the 44th to the 49th from the N-terminus of the M-protein (from the 152nd to 157th from the N-terminus of the L-protein in the case of subtypes adr and adw or from 163rd to 168th in the case of subtype ayw).

Examples of the carriers include particles and microplates of polystyrene, polycarbonate and agarose (Sepharose). The binding of each of the S-, M- and L-proteins to the carrier is performed by contacting an aqueous solution or a buffer containing the protein with the carrier.

In assays for antibodies bound to the carriers, there may be used enzyme-labeled protein A, protein G and secondary antibodies such as anti-human IgG antibody, anti-rabbit IgG antibody and anti-mouse IgG antibody. The enzyme labeling is carried out, for example, by the maleimide method [M. Imamura et al., J. Appl. Biochem. 4, 41 (1982)], by the periodiate oxidation procedure [M. B. Wilson and P. K. Nakane, immunofluorescence and related staining techniques, Knapp, W., Holubar, K. and Wick, G., eds., Elsevier/North Holland Biochemical Press, Amsterdam, pp.215-224 (1978)] or by using a biotinating reagent such as NHS-biotin (biotinyl-N-hydroxy succinimide), sulfo-NHS-biotin or NHS-iminobiotin (Vector Laboratories, Inc.) in combination with an avidinating enzyme. Examples of the enzymes employed include horseradish peroxidase, alkaline phosphatase, beta-galactosidase and glucose oxidase.

The preparation of calibration curves used in the determination can be performed by using sera containing an anti-pre-S1 antibody, an anti-pre-S2 antibody or anti- pre-S antibodies (anti-pre-S1 antibody + anti-pre-S2 antibody). However, commercial immunoglobulin preparations can also be employed. Examples of such preparations include Nichiyaku-HB Globulin (Nippon Seiyaku).

The procedures of the assays for anti-pre-S antibodies according to the present invention will hereinafter be described.

(I) Assay for Anti-Pre-S2 Antibody

Step 1

An S-protein is added to a serum (or a diluted solution thereof) and reacted therewith. By this procedure, anti-S antibodies and antibodies nonspecifically adsorbed by HBV env protein, each of which antibodies is contained in the serum, are absorbed by the S-protein. The serum used is prepared from blood collected by methods known in the art. Also, the IgG fraction prepared from the serum may be used. The reaction of the serum with the S-protein is usually conducted at 4 to 37° C for 1 to 20 hours.

Step 2

An aqueous solution or a buffer containing an M-protein is brought into contact with a carrier, thereby binding the M-protein to the carrier. The reaction is usually conducted at about 4° C for about 20 hours. Then, in order to depress the adsorption of the nonspecific antibodies, it is preferable that an aqueous solution or a buffer containing casein is brought into contact with the carrier. The reaction is usually conducted at about 4° C for about 20 hours.

Step 3

The mixture prepared in Step 1 is brought into contact with the M-protein-binding carrier prepared in Step 2. At this time, only the anti-pre-S2 antibody is bound to the M-protein. The reaction is usually conducted at 4 to 37°C for 1 to 20 hours.

## Step 4

The carrier is separated from the reaction mixture, and an aqueous solution or a buffer containing an enzyme-labeled secondary antibody such as anti-human IgG or anti-human IgM, protein A or protein G is brought into contact with the carrier, thereby reacting it with the anti-pre-S antibody bound to the carrier. The reaction is usually conducted at 4 to 37°C for 1 to 20 hours.

The separation of the carrier from the reaction mixture is easily carried out by filtration or centrifugation when the carrier is composed of particles, or by washing away the reaction mixture with a wash when the carrier is a plate. The washes include water, a sodium chloride solution and a phosphate buffer each of which does not influence enzyme activity, although other washes known to the skilled artisan can be used.

## Step 5

The separation of the carrier from the reaction mixture is carried out in a manner similar to that described in Step 4, and the enzyme bound to the carrier is reacted with a substrate.

Examples of the enzyme substrates include the following:

A. Horseradish peroxidase (HRP)

(1) 5-Aminosalicylic acid - $H_2O_2$
pH at the reaction: 5.6
Assay: absorptiometry ($A_{450}$)
(2) o-Phenylenediamine - $H_2O_2$
pH at the reaction: 4.0
Assay: absorptiometry ($A_{492}$)
(3) Tyramine - $H_2O_2$
pH at the reaction: 8
Assay: fluorophotometry ($\lambda_{ex}$ 320 nm; $\lambda_{em}$ 405 nm)
(4) ABTS [2,2'-azinodi(3-ethylbenzthiazoline)-6'-sulfonic acid] - $H_2O_2$
pH at the reaction: 4.0
Assay: absorptiometry ($A_{405}$' $A_{578}$)

B Alkaline phosphatase

(1) p-Nitrophenol phosphoric acid
pH at the reaction: 8.2
Assay: absorptionmetry ($A_{410}$)
(2) Phenylphosphoric acid-4-aminoantipyrine
pH at the reaction: 10.5
Assay: absorptiometry ($A_{500}$)

C Beta-galactosidase

(1) o-Nitrophenol-$\beta$-D-galactoside
pH at the reaction: 7.8
Assay: absorptiometry ($A_{420}$)
(2) 4-Methylumbelliferyl-$\beta$-D-galactoside
pH at the reaction: 7.8
Assay: fluorophotometry ($\lambda_{ex}$ 330 nm; $\lambda_{em}$ 453 nm: pH at the assay is 10.3)

D. Glucose oxidase

(1) Glucose

Glucose is used as a substrate, and the formed $H_2O_2$ is allowed to luminesce by luminol-peroxidase (pH 8.5) and determined by a chemiluminescence method.

Each of the methods for assaying enzyme activity described above can be performed according to methods well known in the art. As a reaction terminating agent, there is used sodium azide, sulfuric acid or the like.

The amount of anti-pre-S2 antibody contained in the serum can be calculated from a calibration curve prepared by using a standard anti-pre-S2 antibody solution.

(II) Assay for Anti-pre-S1 Antibody

Basic procedures are the same as with the above-mentioned assay for the anti-pre-S2 antibody with the proviso that the S-protein used in Step 1 is substituted for M-protein and the M-protein bound to the carrier in Step 2 is substituted for L-protein.

(III) Assay for Anti-pre-S Antibodies (Anti-pre-S1 Antibody + Anti-pre-S2 Antibody)

Basic procedures are the same as with the above-mentioned assay for the anti-pre-S2 antibody with the proviso that the M-protein bound to the carrier in Step 2 is substituted for L-protein.

The present invention will be described in more detail with the following Examples. It is understood that these examples are not intend to limit the scope of the invention.

Example 1

Assay for Human Anti-pre-S2 Antibody

(1) Preparation of Solid Phase

Purified modified P31 (M-P31c) particles described in J. Biotechnol. 8, 1 (1988) and Japanese Patent Unexamined Publication No. 63-109795 (1988) were added to a buffer of 10 mM $Na_2HPO_4$, 10 mM NaCl and 0.005 % (w/v) thimerosal (pH 8.0) to give a concentration of 20 $\mu$g/ml. Then, 100 $\mu$l of the resulting solution was poured into each well of a microtiter plate (Nunc Inc. Maxisoap), and allowed to stand at 4°C overnight. After washing with three 150 $\mu$l portions of a wash [140 mM NaCl, 0.05 % (w/v) Tween 20], 120 $\mu$l of a blocking buffer [2 % (w/v) casein, 10 mM $Na_2HPO_4 \cdot NaH_2PO_4$ (pH 7.1), 0.005 % (w/v) thimerosal] was added thereto and the plate was allowed to stand at 4°C overnight.

(2) Pre-treatment of Serum

In a Eppendolf tube were placed 200 $\mu$l of a serum collected from a person inoculated with a vaccine obtained from the purified modified M-protein (M-P31c) particles as an immunogen, and were immobilized at 56°C for 30 minutes. According to this procedure, complements are decomposed and nonspecific reactions are reduced. After centrifugation at 15,000 rpm for 5 minutes, 5 $\mu$l of the supernatant was collected and added to 100 $\mu$l of a V8-absorbing antigen solution [60 $\mu$g/ml of M-P31c particles treated with V8 protease, 0.1 % (w/v) Tween 20, 25 % (v/v) fetal calf serum, 140 mM NaCl, 10 mM $Na_2HPO_4/NaH_2PO_4$ (pH 7.1), 0.005 % (w/v) thimerosal] corresponding to a S-protein solution. The resulting mixture was allowed to stand at 37°C for 1 hour or at 4°C for 17 hours.

(3) Primary Reaction

The solid phase was washed with three 150 $\mu$l portions of a wash. Then, each 45 $\mu$l portions/serum of

the mixture prepared in (2) were added to two wells and hermetically sealed, followed by vigorous shaking at room temperature for 1 hour.

According to this procedure, the anti-pre-S2 antibody which has not been adsorbed by the S-protein (M-P31c particles treated with V8 protease) is adsorbed by the solid phase.

### (4) Secondary Reaction

The solid phase was washed with five 300 $\mu$l portions of a wash. Then, 50 $\mu$l of a secondary antibody solution [1/20,000 (v/v) HRP-labeled goat-derived anti-human IgG ($\gamma$) (Kappel), 0.1 % (w/v) Tween 20, 25 % (v/v) fetal calf serum, 140 mM NaCl, 10 mM $Na_2HPO_4/NaH_2PO_4$ (pH 7.1), 0.005 % (w/v) thimerosal] was poured into each well of the solid phase, followed by vigorous shaking at room temperature for 1 hour.

### (5) Color Development

The solid phase was washed with five 300 $\mu$l portions of a wash, and then water was removed therefrom. A HRP substrate solution [24.3 mM citric acid, 51.4 mM $Na_2HPO_4$ (pH 5.0), 0.02 % (w/v) o-phenylenediamine, 0.0013 % (v/v) $H_2O_2$] was added thereto and reacted in the dark at room temperature for 5 minutes. Then, 100 $\mu$l of 2N $H_2SO_4$ was added thereto to terminate the reaction, and $A_{492}$ was measured by Titerteck Multiskan MC (Flow Laboratories).

### (6) Preparation of Calibration Curve

HB Globulin (Nippon Seiyaku) was diluted stepwise with a diluent [0.1 % (w/v) Tween 20, 25 % (v/v) fetal calf serum, 140 mM NaCl, 10 mM $Na_2HPO_4/NaH_2PO_4$ (pH 7.1), 0.005 % (w/v) thimerosal] at concentrations of 1/8, 1/16, 1/32 and 1/64 (v/v) to provide a standard anti-pre-S2 antibody solution. Five $\mu$l of the antibody solution was added to 100 $\mu$l of the V8-absorbing antigen solution, and the color development was performed by the same procedure as with the serum. The results thus obtained are shown in Table 1.

Table 1

| Color Development with Standard Anti-pre-S2 Antibody Solution | | |
|---|---|---|
| Amount of added anti-pre-S2 antibody | Measurement | Change in $A_{492}$ |
| (units[*]/5 $\mu$l of specimen) | ($A_{492}$) | ($\Delta A_{492}$) |
| 0 | 0.103 | 0 |
| 1 | 0.195 | 0.092 |
| 2 | 0.281 | 0.178 |
| 4 | 0.453 | 0.350 |
| 8 | 0.793 | 0.690 |

[*]) One unit corresponds to the amount of the anti-pre-S2 antibody contained in 5 $\mu$l of Nichiyaku HB Globulin diluted to a concentration of 1/64.

The calibration curve obtained from Table 1 is expressed by the following equation when the change in $A_{492}$ ($\Delta A_{492}$) is Y and the amount of the added anti-pre-S2 antibody (unit/5 $\mu$l of specimen) is X:

Y = 0.004 + 0.086X

The correlation coefficient is 0.9999.

Further, by using the calibration curve described above, the anti-pre-S2 antibody titers of human sera were determined before and after the inoculation of a vaccine obtained from the purified modified M-protein (M-P31c) particles as an immunogen. The results are shown in Table 2.

Table 2

| Changes in Anti-Pre-S2 Antibody Titer in Blood before and after Inoculation of Vaccine Containing Purified Modified M-Protein (M-P31c) inoculation* | |
|---|---|
| Vaccine | Amount of antibodies after inoculation on the basis of that before inoculation* (unit/5μl of specimen) |
| A | 9.97 |
| B | 1.31 |
| C | 3.38 |
| D | 6.40 |
| E | 7.62 |

*) The vaccine was inoculated at 0, 4th and 24th weeks, and blood was collected at 28th week by a conventional method.

(7) Establishment of Cutoff Value

The sera of 5 healthy individuals (negative for anti-HBs antibody and negative for anti-pre-S2 antibody) were assayed 16 times an individual as a set of experiments. Four sets of experiments were carried out for 3 days, which totaled 12 sets of experiments. As a result, the standard deviation of the measurements obtained from the total mean value was 0.180 units/5 μl of specimen. From this value, the cutoff value was judged to be 0.460 unit/5 μl of specimen corresponding to 2.58 SD. Namely, a serum exceeding this cutoff value is judged to be positive.

Example 2

Assay for Chimpanzee Anti-pre-S2 Antibody

(1) Preparation of Solid Phase

A solid phase was prepared by a method similar to that described in (1) of Example 1.

(2) Pre-treatment of Serum

The pre-treatment of a serum was conducted by a method similar to that described in (2) of Example 1 except that a chimpanzee was used as the vaccine-inoculating animal.

(3) Primary Reaction

A reaction was conducted by a method similar to that described in (3) of Example 1.

(4) Secondary Reaction

A reaction was conducted by a method similar to that described in (4) of Example 1 except that a solution [1/10000 (v/v) HRP-labeled protein A (Bio RAD), 0.1 % (w/v) Tween 20, 25 % (v/v) fetal calf serum, 140 mM NaCl, 10 mM $Na_2HPO_4/NaH_2PO_4$ (pH 7.1), 0.005 % (w/v) thimerosal] was used as the secondary antibody solution.

### (5) Color Development

Color development was carried out by a method similar to that described in (5) of Example 1.

### (6) Measurements

A calibration curve was obtained in a manner similar to that described in Example 1. The calibration curve is expressed by the following equation when the change in $A_{492}(\Delta A_{492})$ is Y and the amount of the added anti-pre-S2 antibody (unit * /5 $\mu$l of specimen) is X:

$Y = 0.083 + 0.022X$

The correlation coefficient is 0.999.

Further, the amount of the antibodies was calculated from this calibration curve. The results are shown in Table 3.

Table 3

| Changes in Anti-pre-S2 Antibody Titer in Blood before and after Inoculation of Vaccine Containing Purified Modified M-Protein (M-P31c) Particles as Immunogen in Chimpanzee | |
|---|---|
| Chimpanzee | Amount of antibodies after inoculation on the basis of that before inoculation* (unit/5 $\mu$l of specimen) |
| A | 6.21 |
| B | 21.83 |
| C | 52.79 |
| D | 49.23 |
| Control | 0.04 |

*) The vaccine was inoculated at 0, 4th and 8th weeks, and blood was collected at 12th week.

## Example 3

### Assay for Mouse Anti-pre-S1 Antibody

#### (1) Preparation of Solid Phase

A microtiter plate was coated with the purified L-protein (Japanese Patent Application No. 63-95335 (1988)) produced by gene engineering procedures according to (1) of Example 1.

#### (2) Pre-treatment of Serum

*) One unit corresponds to the amount of the anti-pre-S2 antibody contained in 5 $\mu$l of HB Globulin (Nippon Seiyaku) diluted to a concentration of 1/64.

A serum collected from a BALB/c mouse immunized with purified modified L-protein was pre-treated according to (2) of Example 1, with the proviso that an M-P31c-absorbing antigen solution [60 $\mu$g/ml M-P31c particles, 0.1 % (w/v) Tween 20, 25 % (v/v) fetal calf serum, 140 mM NaCl, 10 mM $Na_2HPO_4/NaH_2PO_4$ (pH 7.1), 0.005 % (w/v) thimerosal] was used in place of the V8-absorbing antigen solution.

### (3) Primary Reaction

A reaction was conducted by a method similar to that described in (3) of Example 1.

### (4) Secondary Reaction

A reaction was conducted by a method similar to that described in (4) of Example 1 except that a solution [1/10000 (v/v) HRP-conjugated anti-mouse IgG (H + L) antibody (Bio RAD), 0.1 % (w/v) Tween 20, 25 % (v/v) fetal calf serum, 140 mM NaCl, 10 mM $Na_2HPO_4/NaH_2PO_4$ (pH 7.1), 0.005 % (w/v) thimerosal] was used as the secondary antibody solution.

### (5) Color Development

Color development was carried out by a method similar to that described in (5) of Example 1.

### (6) Measurements

Table 4 shows changes in mouse anti-pre-S1 antibody titer before and after the inoculation of purified LII-protein particles.

Table 4

| Changes in Mouse Anti-pre-S1 Antibody Titer in Blood before and after Inoculation of Vaccine Containing Purified L-Protein Particles as Immunogen | | |
|---|---|---|
| Mouse | Changes in anti-pre-S1 antibody | |
| | Before inoculation | After inoculation* |
| A | 0.465 | 2.000 < |
| B | 0.539 | 2.000 < |
| C | 0.343 | 1.185 |
| D | 0.680 | 2.000 < |
| Control | 0.349 | 0.359 |
| | | $(A_{492})$ |

*) The vaccine was inoculated at 0 week, and blood was collected at the 5th weeks.

The M-P31c particles used in the examples are M-protein particles obtained from Saccharomyces cerevisiae AH22R⁻/pGLD P31-RcT which has been deposited in the Institute for Fermentation, Osaka, Japan (IFO) with the accession number IFO 10206, and in Fermentation Research Institute, Agency of Industrial Science and Technology, Ministry of International Trade and Industry, Japan (FRI) with the accession number FERM BP-1059 under the Budapest Treaty. The L-protein particles are obtained from Saccharomyces cerevisiae AH22R⁻/pGLD LIIP39-RcT which has been deposited in IFO with the accession number IFO 10426, and in FRI with the accession number FERM BP-1747 under the Budapest Treaty.

## Claims

1. An immunoassay for an antibody against a pre-S2 region of a hepatitis B virus surface antigen in a serum which comprises (a) mixing the serum and a hepatitis B virus surface antigen S-protein produced by a unicellular organism, (b) bringing the mixture into contact with a hepatitis B virus surface antigen M-protein produced by a unicellular organism and bound to a carrier, (c) thereby determining the amount of the antibody against the pre-S2 region of the hepatitis B virus surface antigen bound to the carrier.

2. The immunoassay according to claim 1, wherein the unicellular organism is yeast.

3. The immunoassay according to claim 2, wherein the yeast is Saccharomyces cerevisiae.

4. An immunoassay for an antibody against a pre-S1 region of a hepatitis B virus surface antigen in a serum which comprises (a) mixing the serum and a hepatitis B virus surface antigen M-protein produced by a unicellular organism, (b) bringing the mixture into contact with a hepatitis B virus surface antigen L-protein produced by a unicellular organism and bound to a carrier, (c) thereby determining the amount of the antibody against the pre-S1 region of the hepatitis B virus surface antigen bound to the carrier.

5. The immunoassay according to claim 4, wherein the unicellular organism is yeast.

6. The immunoassay according to claim 5, wherein the yeast is Saccharomyces cerevisiae.

7. An immunoassay for an antibody against a pre-S region of a hepatitis B virus surface antigen in a serum which comprises (a) mixing the serum and a hepatitis B virus surface antigen S-protein produced by a unicellular organism, (b) bringing the mixture into contact with a hepatitis B virus surface antigen L-protein produced by a unicellular organism and bound to a carrier, (c) thereby determining the amount of the antibody against the pre-S region of the hepatitis B virus surface antigen bound to the carrier.

8. The immunoassay according to claim 7, wherein the unicellular organism is yeast.

9. The immunoassay according to claim 8, wherein the yeast is Saccharomyces cerevisiae.